# EUROPEAN PATENT SPECIFICATION

(11) **EP 3 549 460 B1**
(45) Date of publication and mention of the grant of the patent: **01.03.2023**
(21) Application number: 17877317.2
(22) Date of filing: 09.05.2017
(51) Int. Cl.: A24F 40/42, A24F 40/49, A24F 40/10

(54) **LIQUID STORAGE ASSEMBLY AND ATOMIZER THEREOF, AND ELECTRONIC CIGARETTE**
FLÜSSIGKEITSSPEICHERANORDNUNG UND ZERSTÄUBER DAFÜR SOWIE ELEKTRONISCHE ZIGARETTE
ENSEMBLE STOCKAGE DE LIQUIDE ET ATOMISEUR ASSOCIÉ, ET CIGARETTE ÉLECTRONIQUE

(30) Priority: 02.12.2016 CN 201611101684
(43) Date of publication of application: 09.10.2019
(73) Proprietor: Changzhou Jwei Intelligent Technology Co., Ltd., Changzhou, Jiangsu 213125 (CN); Joyetech Europe Holding GmbH, 6303 Zug (CH)
(72) Inventor: QIU, Weihua, Jiangsu 213125 (CN)
(74) Representative: Zaboliene, Reda
(86) International application number: PCT/CN2017/083558
(87) International publication number: WO 2018/098996

(56) References cited:
- EP-A1- 3 047 743
- WO-A1-2015/013953
- WO-A1-2016/169063
- CN-A- 104 939 320
- CN-A- 105 686 088
- CN-A- 106 136 328
- CN-A- 106 136 328
- CN-U- 204 907 926
- CN-U- 205 432 130
- US-A1- 2014 182 612
- US-A1- 2015 245 661

## Description

### TECHNICAL FIELD

The present application relates to the technical field of smoking simulation, and more particularly, to a liquid storage assembly, an atomizer, and an electronic cigarette.

### BACKGROUND

Current electronic cigarettes generally have a split type structure, the liquid storage assembly and the atomizing assembly are connected to each other by a pluggable structure or a threaded structure. When the atomizing assembly is detached from the liquid storage assembly, the inlet or outlet hole of the liquid storage assembly for the tobacco liquid is always in an open state, thus the tobacco liquid stored in the liquid storage assembly is easy to leak out. When this kind of electronic cigarette is accessed by children, the liquid storage assembly and the atomizing assembly may be detached from each other by merely a simple operation such as plugging or rotation, thus children may contact or ingest the tobacco liquid by accident, it has a potential safety hazard for children's health.

CN106136328A discloses an electronic cigarette including a cartridge and an atomizer detachably connected to the cartridge. The cartridge includes a cartridge carrier with a liquid storage cavity, a sealing member accommodated at one end of the cartridge carrier, and an adjusting member rotatably mounted at one end of the sealing member near to the atomizer. The sealing member is provided with a second liquid inlet hole in communication with the liquid storage cavity. The adjusting member has a communication portion and a sealing portion. At least one pair of limiting protrusions are axially arranged at one end of the adjusting member near to the atomizer. The cartridge further includes a cartridge base, the cartridge base includes a sleeve. A limiting part is radially arranged at the lower end of the sleeve, and the limiting protrusions abut against the limiting part. The limiting part is convexly provided with a stop protrusion in a direction staggered from the limiting protrusion towards the adjusting member, and the limiting protrusion can be abutted against the stop protrusion by rotating the adjusting member.

### SUMMARY

Accordingly, it is necessary to provide a liquid storage assembly having a children protection function, and an atomizer and an electronic cigarette having the liquid storage assembly, to avoid the contact or ingestion of the tobacco liquid when the electronic cigarette is opened by children.

The technical solution adopted by the present application to solve the problem is set out in the appended set of claims 1-11.

The advantages of the present application are as follows. In the liquid storage assembly, the atomizer or the electronic cigarette provided by the present application, opening or closing of the liquid storage chamber is controlled by rotation of the adjusting member. When the electronic cigarette is detached by children, the adjusting member is driven to rotate and the communication portion is thus isolated from the second liquid inlet hole and the liquid storage chamber is closed, to avoid a leakage of the tobacco liquid and avoid the contact or ingestion of the tobacco liquid by children. It has a simple structure and is convenient to operate.

### BRIEF DESCRIPTION OF THE DRAWINGS

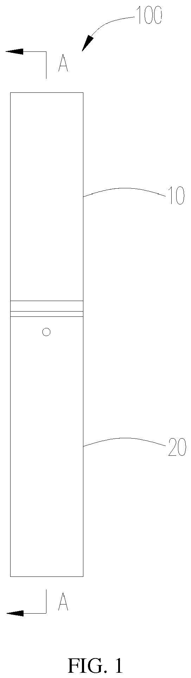

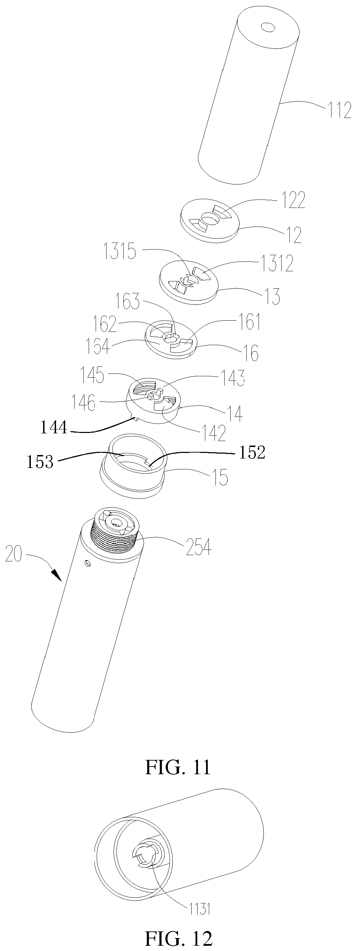

The present application will now be further described with reference to the accompanying drawings and embodiments, wherein the first embodiment shown in FIGS. 1-10 and the second embodiment shown in FIGS. 11-14 are not in accordance with the invention and are present for illustration purposes only.
FIG. 1 is a schematic view of an electronic cigarette according to a first embodiment;
FIG. 2 is an exploded view of the electronic cigarette of FIG. 1;
FIG. 3 is a schematic view of a liquid container of the electronic cigarette of FIG. 2;
FIG. 4 is a schematic view of a sealing member of the electronic cigarette of FIG. 2;
FIG. 5 is a schematic view of an adjusting member of the electronic cigarette of FIG. 2;
FIG. 6 is a schematic view of a lower cover of the electronic cigarette of FIG. 2;
FIG. 7 is a schematic view of an atomizing base of the electronic cigarette of FIG. 2;
FIG. 8 is a schematic view of an atomizing end cover of the electronic cigarette of FIG. 2;
FIG. 9 is a cross-sectional view of the electronic cigarette of FIG. 1 along line A-A (showing the second liquid inlet hole in a closed state);
FIG.10 is another cross-sectional view of the electronic cigarette (showing the second liquid inlet hole in an open state);
FIG. 11 is a partially exploded view of an electronic cigarette according to a second embodiment;
FIG. 12 is a schematic view of a liquid container of the electronic cigarette of FIG. 11;
FIG. 13 is a cross-sectional view of the electronic cigarette of FIG. 11 (showing the liquid storage chamber in a closed state);
FIG. 14 is another cross-sectional view of the electronic cigarette of FIG. 11 (showing the liquid storage chamber in an open state);
FIG. 15 is a schematic view of an electronic cigarette according to a third embodiment;
FIG. 16 is a partially exploded view of the electronic cigarette of FIG. 15;
FIG. 17 is an exploded view of the electronic cigarette of FIG. 15;
FIG. 18 is cross-sectional view of the electronic cigarette of FIG. 15 (showing the liquid storage chamber in a closed state);
FIG. 19 is another cross-sectional view of the electronic cigarette of FIG. 15 (showing the liquid storage chamber in an open state);
FIG. 20 is an exploded view of an electronic cigarette according to a fourth embodiment.

The part names and their reference signs in the figures are:

| | | |
|---|---|---|
| electronic cigarette 100 | liquid storage assembly 10 | atomizing assembly 20 |
| liquid container 11 | pressing member 12 | sealing member 13 |
| adjusting member 14 | lower cover 15 | auxiliary adjusting member 16 |
| housing 21 | battery 22 | atomizing base 23 |
| heating device 24 | atomizing end cover 25 | liquid storage tube 112 |
| exhaust tube 113 | liquid storage chamber 114 | exhaust passage 115 |
| first mounting hole 121 | first liquid inlet hole 122 | positioning hole 123 |
| main body 131 | connecting body 132 | third mounting hole 141 |
| communication portion 142 | sealing portion 143 | abutting portion 144 |
| internal thread 145 | first protrusion 146 | first latching protrusion 151 |
| restricting portion 152 | stopping portion 153 | auxiliary communication portion 161 |
| auxiliary mounting hole 162 | first latching groove 163 | air intake hole 211 |
| supporting base 231 | electrode fixing bracket 232 | liquid absorbing member 241 |
| heating member 242 | second latching protrusion 251 | fourth mounting hole 252 |
| third liquid inlet hole 253 | external thread 254 | resisting portion 1121 |
| second protrusion 1131 | second mounting hole 1311 | second liquid inlet hole 1312 |
| positioning protrusion 1313 | first restricting groove 1314 | second latching groove 1315 |
| air intake groove 2311 | through hole 2312 | elongated groove 2313 |
| second restricting groove 2314 | auxiliary sealing portion 164 | pressure sensor 26 |
| atomizing chamber 255 | electronic cigarette 200 | |
| electronic cigarette 300 | liquid storage assembly 30 | atomizing assembly 40 |
| liquid storage tube 31 | connecting ring 32 | upper cover 33 |
| mouthpiece 34 | lower cover 35 | sealing member 36 |
| adjusting member 37 | atomizing base 41 | atomizing end cover 42 |
| air regulating member 43 | atomizing head 44 | connecting portion 321 |
| annular groove 351 | restricting portion 352 | stopping portion 361 |
| exhaust passage 362 | second liquid inlet hole 363 | abutting portion 371 |
| communication portion 372 | internal thread 373 | first electrode 411 |
| external thread 421 | first internal thread 422 | second electrode 442 |
| engaging portion 431 | first external thread 441 | first sealing member 38 |
| through hole 4421 | liquid storage chamber 311 | |

### DETAILED DESCRIPTION OF EMBODIMENTS

The present application is specifically illustrated with reference to accompanying drawings. The accompanying drawings are schematic views and simplified to show fundamental structures of exemplary embodiments of the present application. Thus, merely the constructions related to the present application are shown. Particularly, the first embodiment shown in FIGS. 1-10 and the second embodiment shown in FIGS. 11-14 are not in accordance with the invention and are present for illustration purposes only.

### First embodiment

Referring to FIG. 1, the present application provides an electronic cigarette 100, the electronic cigarette 100 includes an atomizer (not labeled) and a battery assembly (not labeled) connected to the atomizer. The atomizer includes a liquid storage assembly 10 configured for storing tobacco liquid and an atomizing assembly 20 configured for atomizing the tobacco liquid to form aerosol. The liquid storage assembly 10 and the atomizing assembly 20 are detachably connected. The battery assembly and the atomizing assembly 20 are detachably connected.

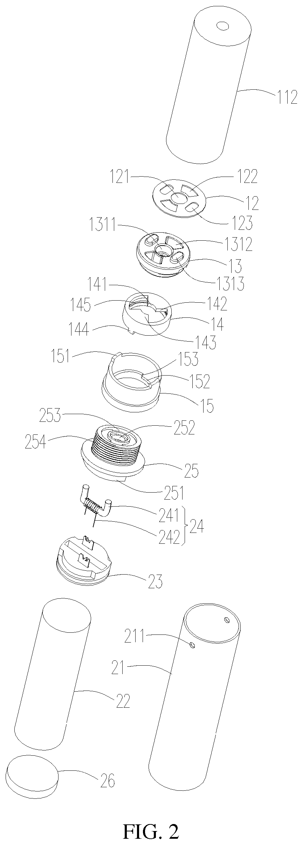

Referring to FIG. 2 and FIG .3, the liquid storage assembly 10 includes a liquid container 11, a pressing member 12, a sealing member 13, an adjusting member 14, and a lower cover 15. The pressing member 12, the sealing member 13 and the adjusting member 14 are received in the liquid container 11. The lower cover 15 is detachably mounted to one end of the liquid container 11 adjacent to the atomizing assembly 20. The adjusting member 14 is rotatable between the sealing member 13 and the lower cover 15.

Further, the liquid container 11 substantially has a tubular shape. The liquid container 11 includes a liquid storage tube 112 having an opening at one end and an exhaust tube 113 received in and extending through the liquid storage tube 112. The exhaust tube 113 is positioned at a center of the liquid storage tube 112. A liquid storage chamber 114 for storing tobacco liquid is formed between an inner wall of the liquid storage tube 112 and an outer wall of the exhaust tube 113. An interior space of the exhaust tube 113 forms an exhaust passage 115 for user to inhale. The user can inhale through the top of the exhaust tube 113.

Further, the liquid storage tube 112 forms a resisting portion 1121 on the inner wall of the liquid storage tube 112 adjacent to the opening, wherein the resisting portion 1121 has an annular shape and extends along a circumferential direction of the liquid storage tube 112.

In the present embodiment, the liquid container 11 can serve as a mouthpiece directly. The user can inhale by keeping one end of the liquid container 11 in his/her mouth. It can be understood that, in an alternative embodiment, a communication member can be connected to the liquid container 11 for communicating with the exhaust tube 113, and the user can inhale by keeping the communication member in his/her mouth.

Referring to FIG. 2, the pressing member 12 substantially has a disc-shaped plate structure. A first mounting hole 121 matching with the exhaust tube 113 is provided at a center of the pressing member 12. The pressing member 12 is further provided with a first liquid inlet hole 122 and a positioning hole 123 around the first mounting hole 121. The pressing member 12 is sleeved on the exhaust tube 113 through the first mounting hole 121. The outer edge of the pressing member 12 resists on the resisting portion 1121 of the liquid storage tube 112 and is connected to the inner wall of the liquid storage tube 112 tightly.

In the present embodiment, the first liquid inlet hole 122 has two and the two first liquid inlet holes 122 are symmetrically arranged about the first mounting hole 121. The positioning hole 123 has two and the two positioning holes 123 are symmetrically arranged about the first mounting hole 121. The first liquid inlet holes 122 and the positioning holes 123 are alternately distributed in a circle. It can be understood that, in an alternative embodiment, the first liquid inlet hole 122 and the positioning hole 123 can each have one or more than two.

In the present embodiment, the pressing member 12 is a plastic circular plate. It can be understood that, in an alternative embodiment, the pressing member 12 can also be made of other hard materials such as stainless steel sheet or ceramic sheet.

Referring also to FIG .4, the sealing member 13 substantially has a cylindrical shape. The sealing member 13 includes a main body 131 and a connecting body 132 protruding from a center of the main body 131. The connecting body 132 is positioned on one end of the main body 131 away from the pressing member 12. The center of the main body 131 is provided with a second mounting hole 1311 which extends through the connecting body 132 and matches with the exhaust tube 113. The main body 131 is provided, around the second mounting hole 1311, with a second liquid inlet hole 1312 corresponding to the first liquid inlet hole 122 and a positioning protrusion 1313 corresponding to the positioning hole 123. The main body 131 is further provided with a first restricting groove 1314 at a circumferential surface of the main body 131. The sealing member 13 is sleeved on one end of the exhaust tube 113 through the second mounting hole 1311. The positioning protrusion 1313 engages in the positioning hole 123. The second liquid inlet hole 1312 is in communication with the first liquid inlet hole 122 of the pressing member 12. The sealing member 13 tightly abuts against the inner wall of the liquid storage tube 112. The second mounting hole 1311 is hermetically connected to the outer wall of the exhaust tube 113.

In the present embodiment, the sealing member 13 is made of rubber. It can be understood that, the sealing member 13 can also be made of silicone, in order for providing a sealing function.

Due to the sealing member 13 made of a soft material, the pressing member 12 is provided to closely contact the sealing member 13 for improving a strength of the sealing member 13, enabling the sealing member 13 not easily to be deformed, thereby avoiding a liquid leakage.

In the present embodiment, the restriction between the sealing member 13 and the pressing member 12 is realized by engagement between the positioning protrusion 1313 and the positioning hole 123. It can be understood that, the pressing member 12 can also be fixed to the sealing member 13 via adhering.

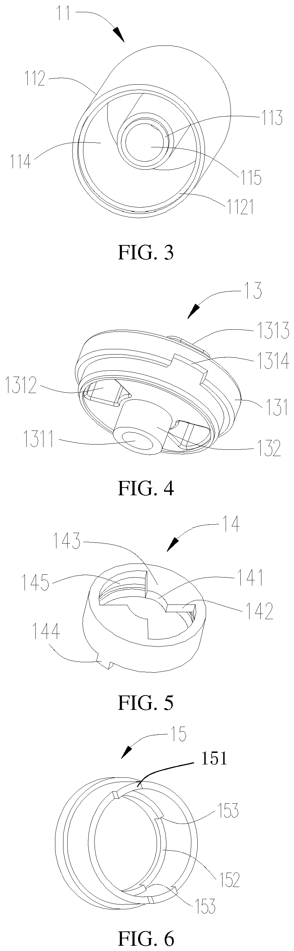

Referring also to FIG. 5, the adjusting member 14 substantially has a tubular shape. A center of the adjusting member 14 is provided with a third mounting hole 141 corresponding to the connecting body 132. The adjusting member 14 is provided with a communication portion 142 and a sealing portion 143 around the third mounting hole 141. The adjusting member 14 is sleeved on the connecting body 132 of the sealing member 13 through the third mounting hole 141. When the adjusting member 14 is rotated, the communication portion 142 and the sealing portion 143 can be brought to be aligned with the second liquid inlet hole 1312 of the sealing member 13 selectively, to thereby open or close the second liquid inlet hole 1312.

The adjusting member 14 is provided with an abutting portion 144. In the present embodiment, the abutting portion 144 is a pair of protrusions disposed oppositely and protruding, along an axial direction of the adjusting member 14, from one end of the adjusting member 14 adjacent to the atomizing assembly 20. The adjusting member 14 is provided with an internal thread 145 on an inner wall of the adjusting member 14.

Referring to FIG. 2 and FIG. 6, the lower cover 15 is substantially a hollow cylindrical shape having openings at two ends. A first latching protrusion 151 protrudes from one end of the lower cover 15 away from the atomizing assembly 20 and matches with the first restricting groove 1314. The inner wall of the lower cover 15 is provided with a restricting portion 152 protruding radially inwardly along a circumferential direction of the lower cover 15, and the restricting portion 152 is provided at one end of the lower cover 15 adjacent to the atomizing assembly 20. The restricting portion 152 has an annular shape. A pair of stopping portions 153 protrudes from the restricting portion 152 towards the adjusting member 14, and the stopping portions 153 are staggered from the abutting portions 144.

After the first latching protrusion 151 of the lower cover 15 engages in the first restricting groove 1314 of the sealing member 13, one end of the adjusting member 14 resists the sealing member 13 axially, and the opposite end of the adjusting member 14 resists the restricting portion 152 axially. Thereby, an axial movement of the adjusting member 14 is restricted.

After the adjusting member 14 is rotated for a certain angle, the abutting portion 144 of the adjusting member 14 will abut against the stopping portion 153 of the lower cover 15 along the circumferential direction. Thereby, a rotation angle of the adjusting member 14 is restricted.

Referring to FIG. 2, the atomizing assembly 20 includes a housing 21, an atomizing base 23 received in the housing 21, a heating device 24 disposed on the atomizing base 23, and an atomizing end cover 25 detachably disposed at an end of the housing 21.

The battery assembly (not labeled) includes a battery 22 received in the housing 21 and a controlling device (not shown) controlling the battery 22 to supply power to the heating device 24.

Further, the housing 21 substantially has a hollow tubular structure having an opening at one end. The tubular wall of the housing 21 is provided with two air intake holes 211 at one end of the housing 21 adjacent to the opening. The two air intake holes 211 are disposed oppositely and are in communication with an interior of the housing 21. It can be understood that, the air intake hole 211 can also have one or more than two.

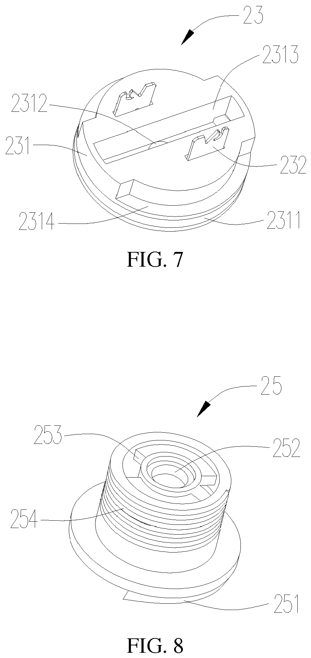

Referring to FIG. 7, the atomizing base 23 has a circular structure. The atomizing base 23 includes a supporting base 231 tightly received in the housing 21 and a pair of electrode fixing brackets 232 disposed on the supporting base 231.

A circumferential surface of the supporting base 231 is provided with an annular air intake groove 2311 in communication with the air intake hole 211. A lower end of the supporting base 231 is provided with a through hole 2312. An upper end of the supporting base 231 is provided with an elongated groove 2313 in communication with the air intake groove 2311 and the through hole 2312. The supporting base 231 is further provided with a second restricting groove 2314 at an outer edge of the upper end of the supporting base 231.

The electrode fixing bracket 232 extends through the supporting base 231. The pair of electrode fixing brackets 232 are respectively electrically connected to a positive electrode and a negative electrode of the battery 22.

Referring to FIG. 2, the heating device 24 includes a liquid absorbing member 241 and a heating member 242 electrically connected to the battery 22. The heating member 242 is wrapped up by the liquid absorbing member 241 or the liquid absorbing member 241 is wrapped up by the heating member 242. The liquid absorbing member 241 is disposed on the upper end of the supporting base 231. The liquid absorbing member 241 can be made of materials having a good absorbing performance, such as glass fiber or natural fiber, etc. Specifically, one end of the heating member 242 is connected to one of the electrode fixing brackets 232, the other end of the heating member 242 is connected to the other one of the electrode fixing brackets 232. The heating member 232 can be made of nichrome wire.

Referring to FIG. 8, the atomizing end cover 25 substantially has a cylindrical structure with flanges and opposite ends thereof are in communication with each other. A second latching protrusion 251 protrudes along an axial direction of the atomizing end cover 25 from one end of the atomizing end cover 25 away from the liquid storage assembly 10, and the second latching protrusion 251 matches with the second restricting groove 2314. A center of the atomizing end cover 25 is provided, corresponding to the connecting body 132, with a fourth mounting hole 252 at one end of the atomizing end cover 25 adjacent to the liquid storage assembly 10. The atomizing end cover 25 is provided with a third liquid inlet hole 253 around the fourth mounting hole 252. An outer wall of the atomizing end cover 25 is provided with an external thread 254 matching with the internal thread 145 of the adjusting member 14.

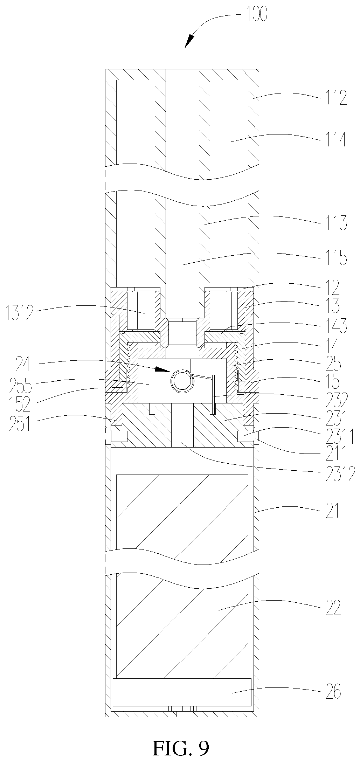

Referring also to FIG. 9, in assembly, the atomizing end cover 25 is mounted on the atomizing base 23 via an engagement between the second latching protrusion 251 and the second restricting groove 2314. An atomizing chamber 255 is formed by a space between the atomizing end cover 25 and the atomizing base 23. The heating device 24 is received in the atomizing chamber 255.

In the present embodiment, the controlling device includes a pressure sensor 26 disposed in the housing 21 and a controller (not shown). One end of the controller is connected to the pressure sensor 26, the other end of the controller is connected to the battery. The pressure sensor 26 is in communication with the air intake hole 211. When the user inhales, a negative pressure is generated in the housing 21. The pressure sensor 26 can detect the change of air pressure in the housing 21, and sends a control signal to the controller, so that the controller controls the battery 22 to supply power to the heating member 241 of the heating device 24, to realize powering on and heating automatically.

It can be understood that, in an alternative embodiment, the controller can also be a switch (not shown) mounted on the outer wall of the housing 21. One end of the switch is connected to an electrode of the battery 22, and the other end of the switch is connected to the electrode fixing bracket 232. The switch can control the ON/OFF state of the battery 22 and the heating device 24. Specifically, the user sucks one puff, the switch is toggled one time, the battery 22 provides power to the heating device 24 one time.

The operation procedure of the electronic cigarette 100 of the first embodiment is described below with reference to the accompanying drawings.

Referring to FIG. 9, at the initial state when the atomizing assembly 20 and the liquid storage assembly 10 of the electronic cigarette 100 are separated, the abutting portion 144 of the adjusting member 14 abuts against one stopping portion 153 of the lower cover 15, the sealing portion 143 of the adjusting member 14 seals the second liquid inlet hole 1312 of the sealing member 13, so that the second liquid inlet hole 1312 is closed, and the tobacco liquid cannot flow out through the second liquid inlet hole 1312 to be atomized.

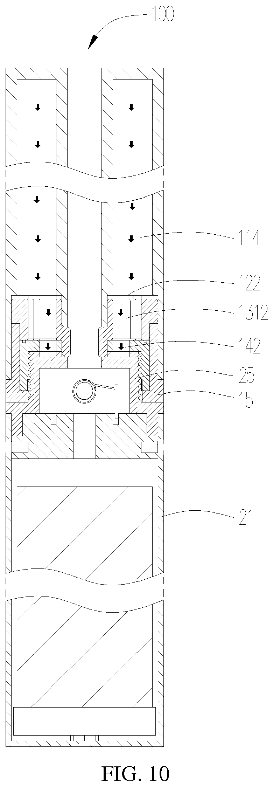

Referring to FIG. 10, in use, the atomizing assembly 20 is rotated along a tightening direction, and the external thread 254 of the atomizing end cover 25 is screwed in along the internal thread 145 of the adjusting member 14. When the screwing force becomes greater than a friction force between the adjusting member 14 and the sealing member 13, the adjusting member 14 is driven to rotate together with the atomizing end cover 25. When the abutting portion 144 is rotated to abut against the other stopping portion 153, the adjusting member 14 stops rotating. At this time, the communication portion 142 of the adjusting member 14 is in communication with the second liquid inlet hole 1312, the liquid storage chamber 114 is thus opened. The tobacco liquid can flow into the atomizing chamber 255 by sequentially passing through the first liquid inlet hole 122, the second liquid inlet hole 1312, the communication portion 142 and the third liquid inlet hole 253, to be absorbed by the liquid absorbing member 241 and atomized by the heating member 242 into aerosol. The arrows in the FIG. 10 indicate a flowing direction of the tobacco liquid.

When detaching, the atomizing assembly 20 is rotated along a loosening direction. Since the screwing force is greater than the friction force between the adjusting member 14 and the sealing member 13, the adjusting member 14 is rotated together with the atomizing end cover 25, until the abutting portion 144 stops rotating by abutting against the stopping portion 153, the adjusting member 14 returns back to the initial state shown in FIG. 9, the sealing portion 143 seals the second liquid inlet hole 1312, the liquid storage chamber 114 is thus closed, the tobacco liquid is blocked and cannot flow out.

When the user inhales, the air enters the electronic cigarette 100 via the air intake hole 211 of the housing 21, and enters the atomizing chamber 255 of the atomizing end cover 25 via the air intake groove 2311 of the atomizing base 23 and the elongated groove 2313 sequentially. The air mixed with the aerosol passes through the connecting body 132 of the sealing member 13, to finally enter the user's mouth through the exhaust passage 115.

It can be understood that, the connecting body 132 can be omitted when the exhaust tube 113 is prolonged, enabling the exhaust tube 113 to extend through the pressing member 12, the sealing member 13, the adjusting member 14 and the atomizing end cover 25, causing the exhaust passage 115 to be in communication with the atomizing chamber 255 directly.

In the electronic cigarette 100 provided by the first embodiment, when the liquid storage assembly 10 is detached and separated from the atomizing assembly 20, the adjusting member 14 is driven to rotate, causing the communication portion 142 to not communicate with the second liquid inlet hole 1312, so that the liquid storage chamber 114 is closed. Even if it is opened by children, a leakage of the tobacco liquid will not occur, to effectively avoid the contact or ingestion of the tobacco liquid by children, thereby having a good safety protection function. Also, it has a simple structure and is convenient to operate.

### Second embodiment

The electronic cigarette 200 of the second embodiment is different from the electronic cigarette 100 of the first embodiment in that, the liquid storage assembly 10 of the electronic cigarette 200 of the second embodiment has a different structure. The liquid storage assembly 10 of the second embodiment is described in detail below.

Referring to FIG. 11, the liquid storage assembly 10 includes a liquid container 11, a pressing member 12, a sealing member 13, an auxiliary adjusting member 16, an adjusting member 14, and a lower cover 15. The liquid container 11, the pressing member 12, the sealing member 13, the auxiliary adjusting member 16 and the adjusting member 14 are received in the liquid container 11. The lower cover 15 is detachably mounted to one end of the liquid container 11 adjacent to the atomizing assembly 20. The auxiliary adjusting member 16 is fixed to the adjusting member 14. The auxiliary adjusting member 16 is driven by the adjusting member 14 to be rotatable between the sealing member 13 and the lower cover 15.

Referring also to FIG. 12, the liquid container 11 substantially has a tubular shape. The liquid container 11 includes a liquid storage tube 112 having an opening at one end and an exhaust tube 113 received in and extending through the liquid storage tube 112. The exhaust tube 113 is positioned at a center of the liquid storage tube 112. The exhaust tube 113 is provided with at least one second protrusion 1131 protruding along an axial direction of the exhaust tube 113 at one end the exhaust tube 113 adjacent to the atomizing assembly 20.

The pressing member 12 substantially has a disc-shaped plate structure. A first mounting hole 121 matching with the exhaust tube 113 is provided at a center of the pressing member 12. The pressing member 12 is further provided with a first liquid inlet hole 122 around the first mounting hole 121.

The sealing member 13 substantially has a circular plate structure. A second mounting hole 1311 corresponding to the exhaust tube 113 is provided at a center of the sealing member 13. The sealing member 13 is provided, around the second mounting hole 1311, with a second liquid inlet hole 1312 corresponding to the first liquid inlet hole 122. The sealing member 13 is provided with a second latching groove 1315 corresponding to the second protrusion 1131. In assembly, the second protrusion 1131 engages in the second latching groove 1315, enabling the sealing member 13 to be fixedly connected to the exhaust tube 113.

The auxiliary adjusting member 16 substantially has a disc-shaped plate structure. A center of the auxiliary adjusting member 16 is provided with an auxiliary mounting hole 162 corresponding to the exhaust tube 113. The auxiliary adjusting member 16 is provided with an auxiliary communication portion 161 and an auxiliary sealing portion 164 around the auxiliary mounting hole 162. When the auxiliary adjusting member 16 is rotated, the auxiliary communication portion 161 is brought to communication with the second liquid inlet hole 1312 or the auxiliary sealing portion 164 is brought to seal the second liquid inlet hole 1312.

Further, the auxiliary adjusting member 16 is provided with at least one first latching groove 163.

The adjusting member 14 substantially has a tubular shape. A center of the adjusting member 14 is provided with a third mounting hole 141 corresponding to the auxiliary mounting hole 162. The adjusting member 14 is provided with a communication portion 142 corresponding to the auxiliary communication portion 161. The adjusting member 14 is provided with a sealing portion 143 corresponding to the auxiliary sealing portion 164. The adjusting member 14 is provided with an abutting portion 144. In the present embodiment, the abutting portion 144 is a pair of protrusions disposed oppositely and protruding, along an axial direction of the adjusting member 14, from one end of the adjusting member 14 adjacent to the atomizing assembly 20. The adjusting member 14 is provided with an internal thread 145 on an inner wall of the adjusting member 14.

Further, the adjusting member 14 is provided with a first protrusion 146 corresponding to the first latching groove 163. In the present embodiment, the first protrusion 146 engages in the first latching groove 163, to fixedly connect the adjusting member 14 and the auxiliary adjusting member 16. It can be understood that, the adjusting member 14 and the auxiliary adjusting member 16 can also be fixedly connected via adhering.

In the electronic cigarette 200 of the second embodiment, the atomizing assembly 20 and the lower cover 15 of the liquid storage assembly 10 have the same structure as the first embodiment, and descriptions thereof are omitted herein for clarity.

In the present embodiment, the sealing member 13 and the auxiliary adjusting member 16 are each made of a ceramic material. The contact surfaces between the sealing member 13 and the auxiliary adjusting member 16 are smooth. When the second liquid inlet hole 1312 is sealed by the auxiliary sealing portion 164, a leakage of the tobacco liquid is avoided. Further, the pressing member 12 closely contacts the end surface of the sealing member 13 away from the adjusting member 14. The pressing member 12 can be made of rubber, and can also be made of silicone. On one hand, the outer circumferential surface of the pressing member 12 closely contacts the inner circumferential surface of the liquid storage tube 112, thereby providing a sealing function. On the other hand, the ceramic material is fragile, when the pressure is large, it may be crushed. Therefore, the pressing member 12 can provide a buffering function, to avoid damage of the sealing member 13 and the auxiliary adjusting member 16.

The operation procedure of the electronic cigarette 200 of the second embodiment is described below with reference to the accompanying drawings.

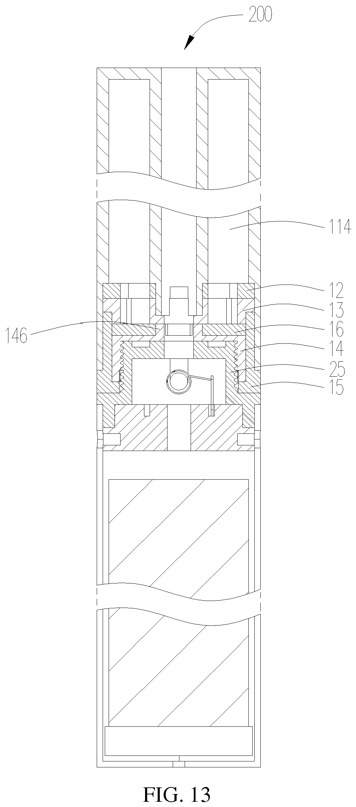

Referring to FIG. 13, at the initial state when the atomizing assembly 20 and the liquid storage assembly 10 of the electronic cigarette 100 are separated, the abutting portion 144 of the adjusting member 14 abuts against one stopping portion 153 of the lower cover 15, the auxiliary sealing portion 164 of the auxiliary adjusting member 16 seals the second liquid inlet hole 1312 of the sealing member 13, so that the second liquid inlet hole 1312 is closed.

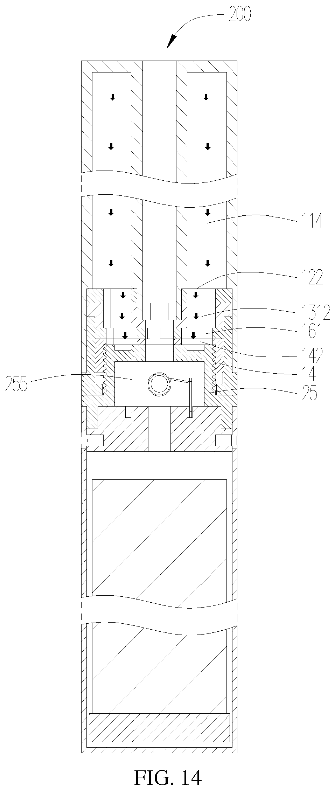

Referring to FIG. 14, in use, the atomizing assembly 20 is rotated along a tightening direction, and the external thread 254 of the atomizing end cover 25 is screwed in along the internal thread 145 of the adjusting member 14. The auxiliary adjusting member 16 is driven by the adjusting member 14 to rotate together with the atomizing end cover 25. When the adjusting member 14 is rotated to cause the abutting portion 144 to abut against the other stopping portion 153, the auxiliary adjusting member 16 stops rotating. At this time, the auxiliary communication portion 161 of the auxiliary adjusting member 16 is in communication with the second liquid inlet hole 1312, the second liquid inlet hole 1312 is thus opened. The tobacco liquid can flow into the atomizing chamber 255 by sequentially passing through the first liquid inlet hole 122, the second liquid inlet hole 1312, the auxiliary communication portion 161, the communication portion 142 and the third liquid inlet hole 253, to be absorbed by the liquid absorbing member 241 and atomized by the heating member 242 into aerosol. The arrows in the FIG. 14 indicate a flowing direction of the tobacco liquid.

In detaching, the atomizing assembly 20 is rotated along a loosening direction. The adjusting member 14 is rotated together with the atomizing end cover 25, until the abutting portion 144 stops rotating by abutting against the stopping portion 153, the adjusting member 14 returns back to the initial state shown in FIG. 12, the auxiliary sealing portion 164 of the auxiliary adjusting member 16 seals the second liquid inlet hole 1312, so that the tobacco liquid is sealed.

Comparing the second embodiment with the first embodiment, a smooth contact is generated between the auxiliary adjusting member 16 and the sealing member 13, the friction force is less, the rotation of the adjusting member is smooth, and the operation is more convenient.

### Third embodiment

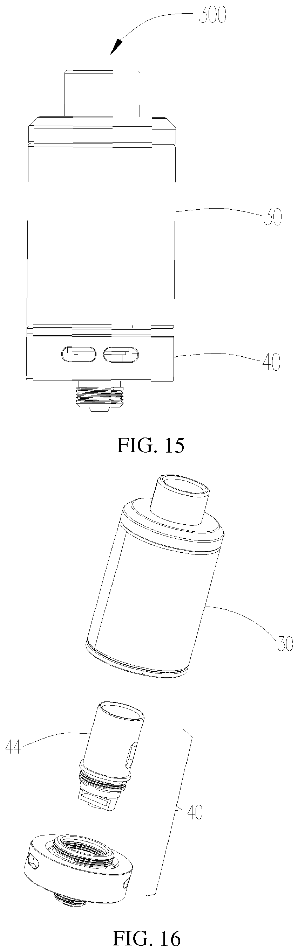

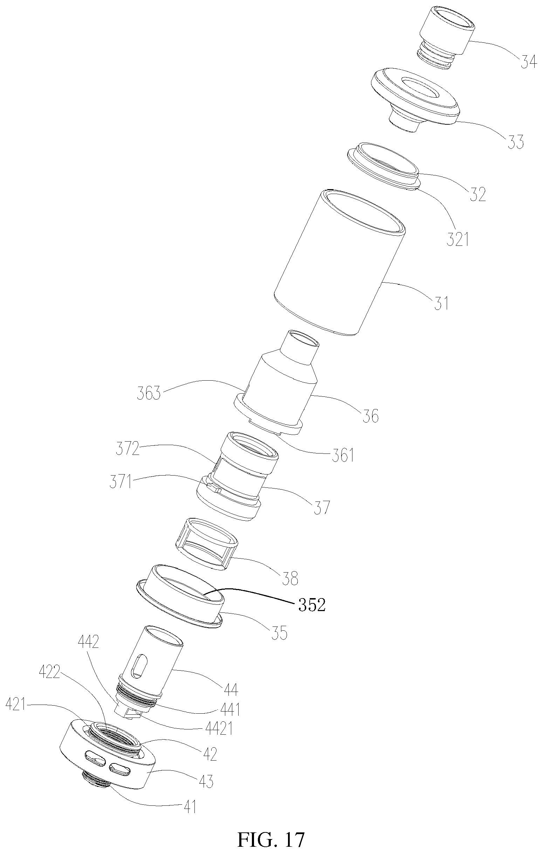

Referring to FIG. 15 and FIG. 17, an electronic cigarette 300 is provided by the third embodiment. The electronic cigarette 300 includes an atomizer (not labeled) and a battery assembly (not shown) detachably connected to the atomizer. The atomizer includes a liquid storage assembly 30 and an atomizing assembly 40 detachably connected to a lower end of the liquid storage assembly 30. The battery assembly is mounted on a lower end of the atomizing assembly 40.

The liquid storage assembly 30 includes a liquid storage tube 31, a connecting ring 32 mounted at an upper end of the liquid storage tube 31, an upper cover 33 mounted on the connecting ring 32, a mouthpiece 34 mounted on the upper cover 33, a lower cover 35 mounted at a lower end of the liquid storage tube 31, a sealing member 36 fixedly connected between the upper cover 33 and the lower cover 35, and an adjusting member 37 rotatably received in the sealing member 36.

The liquid storage tube 31 has a tubular structure. Further, the liquid storage tube 31 is made of transparent material or translucent materials such as glass, for facilitating to observe a residual amount of the tobacco liquid in the liquid storage tube 31.

The connecting ring 32 substantially has an annular structure, the outer wall of the lower end of the connecting ring 32 is provided with a connecting portion 321 along a circumferential direction of the connecting ring 32. The connecting portion 321 is connected to the upper end of the liquid storage tube 31 by an interference fit.

The upper cover 33 substantially has a circular dome structure with a cross-section having an inverted bow shape. The outer edge of the upper cover 33 is connected to an upper end of the connecting ring 32 by an interference fit.

The mouthpiece 34 substantially has a tubular structure having two openings at two ends. A lower end of the mouthpiece 34 is detachably connected to the center of the upper cover 33, and the mouthpiece 34 is in communication with the upper cover 33. It can be understood that, the mouthpiece 34 can be detachably connected to the upper cover 33 through latching, threaded connection, or magnetic connection.

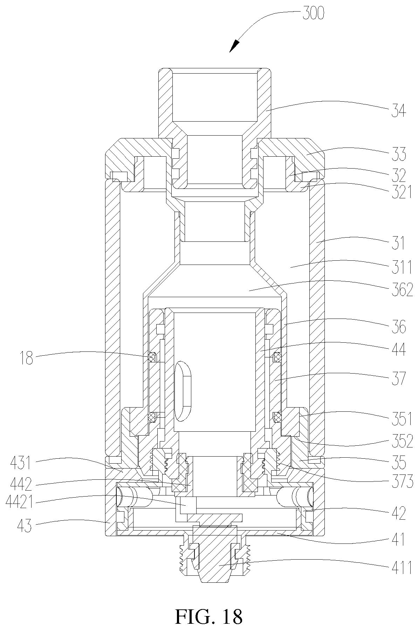

Referring also to FIG. 18, the lower cover 35 substantially has an annular structure. The outer circumferential surface of the lower cover 35 is fixedly connected to the inner circumferential surface of the liquid storage tube 31 by an interference fit. The inner wall of the upper end of the lower cover 35 is provided with an annular groove 351 along a circumferential direction of the lower cover 35, and a restricting portion 352 is formed at the bottom of the annular groove 351.

The sealing member 36 substantially has a hollow tapered tubular structure having openings at two ends. The lower end of the sealing member 36 is provided with a pair of stopping portions 361 disposed oppositely and protruding downwardly from the lower end of the sealing member 36 along an axial direction of the sealing member 36. The upper end of the sealing member 36 is fixedly connected to the lower end of the upper cover 33. The lower end of the sealing member 36 is fixedly connected to the annular groove 351 by an interference fit, causing the stopping portion 361 of the sealing member 36 to abut against the restricting portion 352.

Further, a liquid storage chamber 311 is formed by a space between an inner wall of the liquid storage tube 31 and an outer wall of the sealing member 36. An interior space of the sealing member 36 forms an exhaust passage 362. The sidewall of the sealing member 36 is provided with a second liquid inlet hole 363 in communication with the liquid storage chamber 311.

The adjusting member 37 substantially has a hollow cylindrical structure. The adjusting member 37 is provided with an abutting portion 371. In the present embodiment, the abutting portion 371 is a protrusion formed on a sidewall of the lower end of the adjusting member 37. The abutting portion 371 protrudes radially outwardly from the adjusting member 37 and abuts on the restricting portion 352. During rotation of the adjusting member 37, the outer circumferential surface of the adjusting member 37 closely contacts the inner circumferential surface of the sealing member 36 all the time.

Further, the sidewall of the adjusting member 37 is provided with a communication portion 372 capable of being communicated with the second liquid inlet hole 363. The adjusting member 37 is provided with an internal thread 373 on the inner wall of the lower end of the adjusting member 37.

In order to maintain a sealing effect between the adjusting member 37 and the sealing member 36, and meanwhile to improve a feeling of rotating the adjusting member 37, a first sealing member 38 is positioned between the adjusting member 37 and the sealing member 36. In the present embodiment, the first sealing member 38 is positioned on the outer wall of the adjusting member 37. It can be understood that, the first sealing member 38 can also be positioned on the inner wall of the sealing member 36.

It can be understood that, in an alternative embodiment not shown, the connecting ring 32 can be omitted. At this case, the lower end of the upper cover 33 is directly fixedly connected to the upper end of the liquid storage tube 31.

The atomizing assembly 40 includes an atomizing base 41, an atomizing end cover 42 mounted on the atomizing base 41, an air regulating member 43 rotatably sleeved on the atomizing end cover 42, and an atomizing head 44 detachably connected to an upper end of the atomizing end cover 42.

The atomizing base 41 substantially has a round cup shape. A first electrode 411 is mounted at the center of the atomizing base 41 and is electrically connected to the battery assembly. Further, the atomizing base 41 is provided with a thread to connect with the battery assembly.

The atomizing end cover 42 substantially has an inverted round cup shape. The lower end of the atomizing end cover 42 is fixedly connected to the upper end of the atomizing base 41. An outer wall of the upper end of the atomizing end cover 42 is provided with an external thread 421 matching with the internal thread 373. The inner wall of the upper end of the atomizing end cover 42 is provided with a first internal thread 422.

Further, the sidewall of the atomizing end cover 42 is provided with a first air intake hole (not labeled).

The air regulating member 43 substantially has an annular structure. The upper end of the air regulating member 43 forms an engaging portion 431 extending inwardly along a circumferential direction of the air regulating member 43. The engaging portion 431 abuts on the atomizing end cover 42 to prevent a disengagement of the air regulating member 43.

Further, the sidewall of the air regulating member 43 is provided with a second air intake hole (not labeled) corresponding to the first air intake hole. The air regulating member 43 can be rotated to enable the second air intake hole to communicate with the first air intake hole or enable the air regulating member 43 to seal the first air intake hole, thereby controlling the amount of air intake.

The atomizing head 44 is provided with a first external thread 441 matching with the first internal thread 422. A second electrode 442 is further assembled to the lower end of the atomizing head 44. The lower end of the second electrode 442 is electrically connected to the first electrode 411. The upper end of the second electrode 442 is electrically connected to the heating member (not shown) in the atomizing head 44. The heating member is heated to atomize the tobacco liquid flowing into the atomizing head 44 from the liquid storage chamber 311, causing the tobacco liquid to become aerosol for the user to inhale.

Further, the second electrode 442 is provided with a through hole 4421 in communication with the first air intake hole and the interior chamber of the atomizing head 44. The external air can flow into the interior chamber of the atomizing head 44 by sequentially passing through the second air intake hole, the first air intake and the through hole 4421, to mix with the aerosol. The mixed gas reaches the user's mouth by sequentially passing through the exhaust passage 362, the upper cover 33 and the mouthpiece 34.

Further, an insulating member (not labeled) is positioned between the atomizing base 41 and the first electrode 411, and between the atomizing head 44 and the second electrode 442, for providing an insulation function.

The operation procedure of the electronic cigarette 300 is described below with reference to the accompanying drawings.

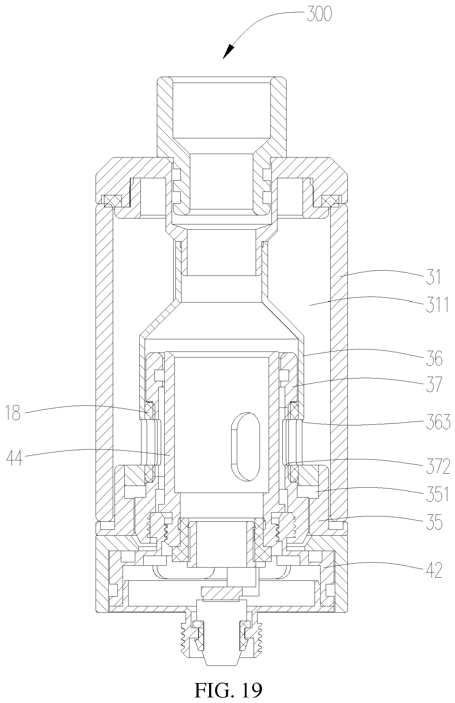

Referring to FIG. 19, when the liquid storage assembly 30 and the atomizing assembly 40 are assembled, firstly, the atomizing head 44 is connected to the atomizing end cover 42 via threads, and then the atomizing assembly 40 is rotated along a tightening direction, the atomizing end cover 42 is gradually screwed into the adjusting member 37. When the screwing force becomes greater than a friction force between the adjusting member 37 and the sealing member 36, the adjusting member 37 is driven to rotate together with the atomizing end cover 42. When the abutting portion 371 is rotated to abut against the stopping portion 361, the adjusting member 37 stops rotating. At this time, the second liquid inlet hole 363 is in communication with the communication portion 372, the liquid storage chamber 311 is thus opened. The tobacco liquid can flow into the interior chamber of the atomizing head 44 by sequentially passing through the second liquid inlet hole 363 and the communication portion 372, to be atomized into aerosol. When the atomizing assembly 40 is continuously rotated to tighten, the atomizing end cover 42 and the adjusting member 37 are mounted in position.

Referring to FIG. 18, when the liquid storage assembly 30 and the atomizing assembly 40 are detached, the atomizing assembly 40 is rotated along a loosening direction. Since the screwing force is initially greater than a friction force between the adjusting member 37 and the sealing member 36, the adjusting member 37 is rotated together with the atomizing end cover 42, until the abutting portion 371 stops rotating by abutting against the other stopping portion 361. At this time, the second liquid inlet hole 363 is isolated from the communication portion 372, the liquid storage chamber 311 is thus closed, and the tobacco liquid cannot flow out. When the atomizing assembly 40 is continuously rotated to loosen, the atomizing end cover 42 and the adjusting member 37 are separated completely.

In the electronic cigarette 300 provided by the third embodiment, when the atomizing assembly 40 is detached and separated from the liquid storage assembly 30, the adjusting member 37 is driven to rotate, causing the communication portion 372 to not communicate with the second liquid inlet hole 363, so that the liquid storage chamber 311 is closed and the tobacco liquid cannot flow out. Even if the electronic cigarette 300 is opened by children, a leakage of the tobacco liquid will not occur, to avoid the contact or ingestion of the tobacco liquid by children, thereby having a good safety protection function.

### Fourth embodiment

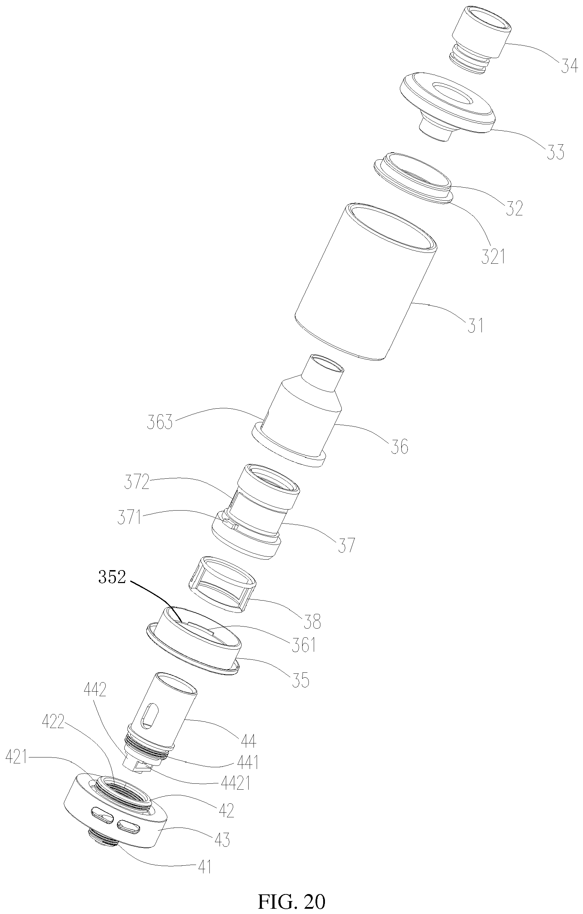

Referring to FIG. 20, the main difference between the electronic cigarette provided by the fourth embodiment and the electronic cigarette 300 provided by the third embodiment lies in that, the stopping portion 361 has two, and the two stopping portions 361 protrude from the restricting portion 352 and are disposed oppositely. In use, the abutting portion 371 can resist the restricting portion 352 axially and resist the stopping portion 361 circumferentially. The working principle is the same as that in the third embodiment, and is herein omitted for clarity.

In the electronic cigarette of the fourth embodiment, the stopping portion 361 is provided on the lower cover 35. When the liquid storage assembly 30 and the atomizing assembly 40 are detached and separated, the liquid storage chamber 311 can also be sealed, thereby having a good safety protection function.

The embodiments described above are merely preferred embodiments, but not intended to limit the present application. Any modifications, alternatives or improvements can be made within the protection scope of the present application, as defined in the appended claims.

## Claims

1. A liquid storage assembly (30) comprising a liquid storage tube (31) having a liquid storage chamber (311), a sealing member (36) and an adjusting member (37) received in the liquid storage tube (31), and a lower cover (35) arranged at one end of the liquid storage tube (31), wherein the adjusting member (37) is rotatable relative to the sealing member (36), the sealing member (36) is provided with a second liquid inlet hole (363) in communication with the liquid storage chamber (311), the adjusting member (37) is provided with a communication portion (372) corresponding to the second liquid inlet hole (363), the lower cover (35) or the sealing member (36) is provided with a stopping portion (361), the adjusting member (37) is provided with an abutting portion (371) capable of abutting against the stopping portion (361); when the liquid storage assembly (30) is detached, the adjusting member (37) is rotated to a position at which the abutting portion (371) abuts against the stopping portion (361) and the adjusting member (37) seals the second liquid inlet hole (363) so as to close the liquid storage chamber (311), **characterized in that** the liquid storage assembly (30) further comprises an upper cover (33) mounted at an upper end of the liquid storage tube (31), the lower cover (35) is mounted at a lower end of the liquid storage tube (31), the sealing member (36) is fixedly connected between the upper cover (33) and the lower cover (35), the adjusting member (37) is rotatably received in the sealing member (36), the liquid storage chamber (311) is formed by a space between an inner wall of the liquid storage tube (31) and an outer wall of the sealing member (36), the second liquid inlet hole (363) is provided in the sidewall of the sealing member (36), the communication portion (372) capable of being communicated with the second liquid inlet hole (363) is provided in the sidewall of the adjusting member (37).

2. The liquid storage assembly (30) according to claim **1,** wherein an inner wall of the lower cover (35) is provided with a restricting portion (352), the stopping portion (361) has two and the two stopping portions (361) protrude from the restricting portion (352) and are disposed oppositely, the abutting portion (371) abuts against the restricting portion (352) axially and abuts against the stopping portions (361) circumferentially.

3. The liquid storage assembly (30) according to claim **1,** wherein an inner wall of the lower cover (35) is provided with a restricting portion (352), the stopping portion (361) has two and the two stopping portions (361) protrude downwardly from a lower end of the sealing member (36) along an axial direction of the sealing member (36), the abutting portion (371) abuts against the restricting portion (352) axially and abuts against the stopping portions (361) circumferentially.

4. The liquid storage assembly (30) according to claim **3,** wherein the inner wall of the upper end of the lower cover (35) is provided with an annular groove (351) along a circumferential direction of the lower cover (35), the restricting portion (352) is formed at the bottom of the annular groove (351), the upper end of the sealing member (36) is fixedly connected to the lower end of the upper cover (33), the lower end of the sealing member (36) is fixedly connected to the annular groove (351), causing the stopping portion (361) of the sealing member (36) to abut against the restricting portion (352).

5. The liquid storage assembly (30) according to claim **1**, wherein the abutting portion (371) is a protrusion formed on a sidewall of the lower end of the adjusting member (37), the abutting portion (371) protrudes radially outwardly from the adjusting member (37).

6. The liquid storage assembly (30) according to claim **1**, wherein during rotation of the adjusting member (37), the outer circumferential surface of the adjusting member (37) closely contacts the inner circumferential surface of the sealing member (36) all the time.

7. The liquid storage assembly (30) according to claim **1**, wherein the communication portion (372) is a through hole extending through the adjusting member (37).

8. An atomizer comprising the liquid storage assembly (30) according to either one of claims **1** to 7 and an atomizing assembly (40) detachably connected to the liquid storage assembly (30).

9. The atomizer according to claim **8**, wherein the atomizing assembly (40) comprises an atomizing base (41) and an atomizing end cover (42) mounted on the atomizing base (41), the adjusting member (37) is provided with an internal thread (373) on an inner wall of the adjusting member (37), an outer wall of the atomizing end cover (42) is provided with an external thread (421) matching with the internal thread (373), the external thread (421) is screwed with the internal thread (373), when a screwing force between the external thread (421) and the internal thread (373) is greater than a friction force between the adjusting member (37) and the sealing member (36), the adjusting member (37) is driven to rotate together with the atomizing end cover (42).

10. The atomizer according to claim **9**, wherein the inner wall of the upper end of the atomizing end cover (42) is provided with a first internal thread (422), the lower end of the atomizing head (44) is provided with a first external thread (441) matching with the first internal thread (422), the upper end of the atomizing head (44) is received in the adjusting member (37).

11. An electronic cigarette comprising the atomizer according to claim **8** and a battery assembly connected to the atomizer.

## Patentansprüche

1. Flüssigkeitsspeicheranordnung (30), umfassend ein Flüssigkeitsspeicherrohr (31), das eine Flüssigkeitsspeicherkammer (311), ein Dichtungselement (36) und ein Einstellelement (37) aufweist, die in dem Flüssigkeitsspeicherrohr (31) aufgenommen sind, und eine untere Abdeckung (35), die an einem Ende des Flüssigkeitsspeicherrohrs (31) angeordnet ist, wobei das Einstellelement (37) relativ zu dem Dichtungselement (36) drehbar ist, wobei das Dichtungselement (36) mit einem zweiten Flüssigkeitseinlassloch versehen ist (363), das in Verbindung mit der Flüssigkeitsspeicherkammer (311) steht, das Einstellelement (37) mit einem Verbindungsabschnitt (372) versehen ist, der dem zweiten Flüssigkeitseinlassloch (363) entspricht, die untere Abdeckung (35) oder das Dichtungselement (36) mit einem Stoppabschnitt (361) versehen ist, das Einstellelement (37) mit einem Anschlag (371) versehen ist, der in der Lage ist, gegen den Stoppabschnitt (361) anzustoßen; wenn die Flüssigkeitsspeicheranordnung (30) abgenommen wird, wird das Einstellelement (37) in eine Position gedreht, in der der Anschlag (371) gegen den Stoppabschnitt (361) stößt, und das Einstellelement (37) das zweite Flüssigkeitseinlassloch (363) abdichtet, um die Flüssigkeitsspeicherkammer (311) zu schließen, **dadurch gekennzeichnet, dass** die Flüssigkeitsspeicheranordnung (30) ferner eine obere Abdeckung (33) umfasst, die am oberen Ende des Flüssigkeitsspeicherrohrs (31) montiert ist, die untere Abdeckung (35) am unteren Ende des Flüssigkeitsspeicherrohrs (31) montiert ist, das Dichtungselement (36) fest zwischen der oberen Abdeckung (33) und der unteren Abdeckung (35) verbunden ist, das Einstellelement (37) drehbar im Dichtungselement (36) aufgenommen ist, die Flüssigkeitsspeicherkammer (311) durch einen Raum zwischen der Innenwand des Flüssigkeitsspeicherrohrs (31) und der Außenwand des Dichtungselements (36) gebildet ist, das zweite Flüssigkeitseinlassloch (363) in der Seitenwand des Dichtungselements (36) vorgesehen ist, der Verbindungsabschnitt (372), der mit dem zweiten Flüssigkeitseinlassloch (363) verbunden werden kann, in der Seitenwand des Einstellelements (37) vorgesehen ist.

2. Flüssigkeitsspeicheranordnung (30) nach Anspruch 1, wobei die Innenwand der unteren Abdeckung (35) mit einem Begrenzungsabschnitt (352) versehen ist, der Stoppabschnitt (361) zwei Stoppabschnitte aufweist und die zwei Stoppabschnitte (361) vom Begrenzungsabschnitt (352) vorstehen und gegenüberliegend angeordnet sind, der Anschlag (371) axial an dem Begrenzungsabschnitt (352) und in Umfangsrichtung an den Stoppabschnitten (361) anliegt.

3. Flüssigkeitsspeicheranordnung (30) nach Anspruch 1, wobei die Innenwand der unteren Abdeckung (35) mit einem Begrenzungsabschnitt (352) versehen ist, der Stoppabschnitt (361) zwei Stoppabschnitte aufweist und die zwei Stoppabschnitte (361) vom unteren Ende des Dichtungselements (36) entlang der axialen Richtung des Dichtungselements (36) nach unten vorstehen, der Anschlag (371) axial an dem Begrenzungsabschnitt (352) und in Umfangsrichtung an den Stoppabschnitten (361) anliegt.

4. Flüssigkeitsspeicheranordnung (30) nach Anspruch 3, wobei die Innenwand des oberen Endes der unteren Abdeckung (35) mit einer ringförmigen Nut (351) entlang der Umfangsrichtung der unteren Abdeckung (35) versehen ist, der Begrenzungsabschnitt (352) am Boden der ringförmigen Nut (351) ausgebildet ist, das obere Ende des Dichtungselements (36) fest mit dem unteren Ende der oberen Abdeckung (33) verbunden ist, das untere Ende des Dichtungselements (36) fest mit der ringförmigen Nut (351) verbunden ist, was bewirkt, dass der Stoppabschnitt (361) des Dichtungselements (36) gegen den Begrenzungsabschnitt (352) stößt.

5. Flüssigkeitsspeicheranordnung (30) nach Anspruch 1, wobei der Anschlag (371) ein Vorsprung ist, der an einer Seitenwand des unteren Endes des Einstellelements (37) ausgebildet ist, wobei der Anschlag (371) radial nach außen vom Einstellelement (37) vorsteht.

6. Flüssigkeitsspeicheranordnung (30) nach Anspruch 1, wobei während der Drehung des Einstellelements (37) die Außenumfangsfläche des Einstellelements (37) die ganze Zeit eng mit der Innenumfangsfläche des Dichtungselements (36) in Kontakt steht.

7. Flüssigkeitsspeicheranordnung (30) nach Anspruch 1, wobei der Verbindungsabschnitt (372) ein Durchgangsloch ist, das sich durch das Einstellelement (37) erstreckt.

8. Zerstäuber, umfassend die Flüssigkeitsspeicheranordnung (30) nach einem der Ansprüche 1 bis 7 und eine Zerstäuberanordnung (40), die abnehmbar mit der Flüssigkeitsspeicheranordnung (30) verbunden ist.

9. Zerstäuber nach Anspruch 8, wobei die Zerstäuberanordnung (40) eine Zerstäuberbasis (41) und eine an der Zerstäuberbasis (41) angebrachte Zerstäuberendabdeckung (42) umfasst, wobei das Einstellelement (37) mit einem Innengewinde (373) an der Innenwand des Einstellelements (37) versehen ist, die Außenwand der Zerstäuberendabdeckung (42) mit einem zum Innengewinde (373) passenden Außengewinde (421) versehen ist, wobei das Außengewinde (421) mit dem Innengewinde (373) verschraubt wird, wenn das Schneckendrehmoment zwischen dem Außengewinde (421) und dem Innengewinde (373) größer als die Reibungskraft zwischen dem Einstellelement (37) und dem Dichtungselement (36) ist, wobei das Einstellelement (37) angetrieben wird, um sich zusammen mit der Zerstäuberendabdeckung (42) zu drehen.

10. Zerstäuber nach Anspruch 9, wobei die Innenwand des oberen Endes der Zerstäuberendabdeckung (42) mit einem ersten Innengewinde (422) versehen ist, das untere Ende des Zerstäuberkopfes (44) mit einem ersten Außengewinde (441) versehen ist, das mit dem ersten Innengewinde (422) zusammenpasst, das obere Ende des Zerstäuberkopfes (44) in dem Einstellelement (37) aufgenommen ist.

11. Elektronische Zigarette, die den Zerstäuber nach Anspruch 8 und eine mit dem Zerstäuber verbundene Batterieanordnung umfasst.

## Revendications

1. Un ensemble de stockage de liquide (30) comprenant un tube de stockage de liquide (31) ayant un réservoir de stockage de liquide (311), un élément d'étanchéité (36) et un élément de réglage (37) reçus dans le tube de stockage de liquide (31), dans lequel l'élément de réglage (37) peut tourner par rapport à l'élément d'étanchéité (36), l'élément d'étanchéité (36) est pourvu d'un second trou d'entrée de liquide (363) en communication avec le réservoir de stockage de liquide (311), l'élément de réglage (37) est pourvu d'une partie de communication (372) correspondant au second trou d'entrée de liquide (363), le couvercle inférieur (35) ou l'élément d'étanchéité (36) est pourvu d'une partie d'arrêt (361), l'élément de réglage (37) est muni d'une partie attenante (371) capable de venir en butée contre la partie d'arrêt (361) ; lorsque l'ensemble de stockage de liquide (30) est détaché, l'élément de réglage (37) est tourné sur une position dans laquelle la partie attenante (371) bute contre la partie d'arrêt (361) et l'élément de réglage (37) scelle le second trou d'entrée de liquide (363) de manière à fermer le réservoir de stockage de liquide (311), **caractérisé en ce que** l'ensemble de stockage de liquide (30) comprend en outre un couvercle supérieur (33) monté à une extrémité supérieure du tube de stockage de liquide (31), le couvercle inférieur (35) est monté à une extrémité inférieure du tube de stockage de liquide (31), l'élément d'étanchéité (36) est relié fixement entre le couvercle supérieur (33) et le couvercle inférieur (35), l'élément de réglage (37) est reçu de manière rotative dans l'élément d'étanchéité (36), le réservoir de stockage de liquide (311) est formé par un espace entre une paroi intérieure du tube de stockage de liquide (31) et une paroi extérieure de l'élément d'étanchéité (36), le second trou d'entrée de liquide (363) est prévu dans la paroi latérale de l'élément d'étanchéité (36), la partie de communication (372) pouvant être mise en communication avec le second trou d'entrée de liquide (363) est prévue dans la paroi latérale de l'élément de réglage (37).

2. L'ensemble de stockage de liquide (30) selon la revendication 1, dans laquelle une paroi intérieure du couvercle inférieur (35) est pourvue d'une partie de restriction (352), la partie d'arrêt (361) en comporte deux et les deux parties d'arrêt (361) dépassent de la partie de restriction (352) et sont disposées de manière opposée, la partie attenante (371) vient en butée contre la partie de restriction (352) axialement et vient en butée contre les parties d'arrêt (361) circonférentiellement.

3. L'ensemble de stockage de liquide (30) selon la revendication 1, dans laquelle une paroi intérieure du couvercle inférieur (35) est pourvue d'une partie de restriction (352), la partie d'arrêt (361) en comporte deux et les deux parties d'arrêt (361) dépassent vers le bas d'une extrémité inférieure de l'élément d'étanchéité (36) le long d'une direction axiale de l'élément d'étanchéité (36), la partie attenante (371) bute contre la partie de restriction (352) axialement et bute contre les parties d'arrêt (361) circonférentiellement.

4. L'ensemble de stockage de liquide (30) selon la revendication 3, dans laquelle la paroi intérieure de l'extrémité supérieure du couvercle inférieur (35) est pourvue d'une rainure annulaire (351) le long d'une direction circonférentielle du couvercle inférieur (35), la partie de restriction (352) est formée au fond de la rainure annulaire (351), l'extrémité supérieure de l'élément d'étanchéité (36) est relié de manière fixe à l'extrémité inférieure du couvercle supérieur (33), l'extrémité inférieure de l'élément d'étanchéité (36) est relié de manière fixe à la rainure annulaire (351), amenant la partie d'arrêt (361) de l'élément d'étanchéité (36) à buter contre la partie de restriction (352).

5. L'ensemble de stockage de liquide (30) selon la revendication 1, dans laquelle la partie attenante (371) est une saillie formée sur une paroi latérale de l'extrémité inférieure de l'élément de réglage (37), la partie attenante (371) fait saillie radialement vers l'extérieur depuis l'élément de réglage (37).

6. L'ensemble de stockage de liquide (30) selon la revendication 1, dans laquelle pendant la rotation de l'élément de réglage (37), la surface circonférentielle extérieure de l'élément de réglage (37) est étroitement en contact avec la surface circonférentielle intérieure de l'élément d'étanchéité (36).

7. L'ensemble de stockage de liquide (30) selon la revendication 1, dans laquelle la partie de communication (372) est un trou traversant s'étendant à travers l'élément de réglage (37).

8. Un atomiseur comprenant l'ensemble de stockage de liquide (30) selon n'importe laquelle des revendications 1 à 7 et un ensemble d'atomisation (40) relié de manière amovible à l'ensemble de stockage de liquide (30).

9. L'atomiseur selon la revendication 8, dans laquelle l'ensemble d'atomisation (40) comprend une base d'atomisation (41) et un couvercle d'extrémité d'atomisation (42) monté sur la base d'atomisation (41), l'élément de réglage (37) est pourvu d'un filetage intérieur (373) sur une paroi intérieure de l'élément de réglage (37), une paroi extérieure du couvercle d'extrémité d'atomisation (42) est pourvue d'un filetage extérieur (421) correspondant au filetage intérieur (373), le filetage extérieur (421) est vissé avec le filetage intérieur (373), lorsqu'une force de vissage entre le filetage externe (421) et le filetage interne (373) est supérieure à une force de friction entre l'élément de réglage (37) et l'élément d'étanchéité (36), l'élément de réglage (37) est entraîné pour tourner conjointement avec le couvercle d'extrémité d'atomisation (42).

10. L'atomiseur selon la revendication 9, dans laquelle la paroi interne de l'extrémité supérieure du couvercle d'extrémité d'atomisation (42) est pourvue d'un premier filetage interne (422), l'extrémité inférieure de la tête d'atomisation (44) est pourvue d'un premier filetage externe (441) correspondant au premier filetage interne (422), l'extrémité supérieure de la tête d'atomisation (44) est reçue dans l'élément de réglage (37).

11. Une cigarette électronique comprenant l'atomiseur selon la revendication 8 et un ensemble de batterie connecté à l'atomiseur.
